# EUROPEAN PATENT APPLICATION

(11) **EP 4 748 375 A1**
(43) Date of publication of application: **27.05.2026**
(21) Application number: 24844611.4
(22) Date of filing: 10.07.2024
(51) Int. Cl.: A61K 31/55, A61K 31/4355, A61P 11/00, A61P 1/16

(54) **PHARMACEUTICAL COMPOSITION AND METHOD FOR TREATING ORGAN FIBROSIS-RELATED DISEASES**

(30) Priority: 21.07.2023 CN 202310905545
(71) Applicant: Shionogi China Co., Ltd., Shanghai 200232 (CN)
(72) Inventor: YAMAKAWA, Hidekuni, Shanghai 200232 (CN)
(74) Representative: Ghirardi, Valeria
(86) International application number: PCT/CN2024/104587
(87) International publication number: WO 2025/020917

(57) **Abstract**

Disclosed herein are pharmaceutical compositions and methods for treating organ fibrosis related diseases. The pharmaceutical compositions and methods of the present invention comprise mirtazapine or a pharmaceutically acceptable salt thereof, or a combination of mirtazapine or a pharmaceutically acceptable salt thereof and noscapine or a pharmaceutically acceptable salt thereof, which can effectively inhibit organ fibrosis lesions in patients, thereby treating organ fibrosis related diseases.

## Description

### Technical Field

The present invention belongs to the field of medicine and relates to pharmaceutical compositions and methods for treating organ fibrosis related diseases, particularly to pharmaceutical compositions and methods for treating fibrotic interstitial lung diseases.

### Background

Organ fibrosis is a pathological change characterized by increased fibrous connective tissue and decreased parenchymal cells in organ tissues, and it is a common pathological feature of various chronic diseases. Fibrosis of parenchymal organs such as the heart, liver, lungs, and kidneys is a major cause of organ failure, disability, and death in patients. However, there is still a lack of therapeutic drugs specifically targeting fibrosis to date (Han Min, et al. Research Progress and Key Scientific Issues of Organ Fibrosis [J]. Chinese Journal of Pathophysiology, 2018, 34(08): 1518-1526). Pulmonary fibrosis (PF) is a chronic, progressive interstitial lung disease and one of the most common clinical respiratory diseases. Its pathogenesis remains unclear, and currently, there is still a lack of specific drugs and therapeutic methods in clinical practice (Zhou Shiqin, et al. Related Molecular Mechanisms and Therapeutic Status of Pulmonary Fibrosis [J]. Chinese Journal of Clinical Pharmacology and Therapeutics, 2022, 27(10): 1133-1147). Fibrosing interstitial lung diseases (FILDs) are a group of heterogeneous diseases with different etiologies, treatment approaches, and a range of disease manifestations. They are characterized by the pathological finding of fibrosis or the presence of honeycombing and/or traction bronchiectasis on high-resolution computed tomography (HRCT) (Rajan SK, et al. Progressive pulmonary fibrosis: an expert group consensus statement. Eur Respir J. 2023 Mar 30; 61(3): 2103187). Fibrosing interstitial lung diseases include idiopathic pulmonary fibrosis (IPF) and progressive fibrosing interstitial lung diseases (PF-ILDs). Progressive fibrosing interstitial lung diseases are a group of refractory diseases with a high mortality rate, with a 5-year survival rate of less than 50%, while idiopathic pulmonary fibrosis (IPF) is a type of disease characterized primarily by pulmonary fibrosis (Zhou Ping, A Brief Discussion on the Pathogenesis of Pulmonary Fibrosis [J]. Smart Healthcare, 2022, 8(24): 110-113). To date, only two drugs have been approved for the treatment of idiopathic pulmonary fibrosis: pirfenidone and nintedanib. However, nintedanib and pirfenidone can cause side effects such as loss of appetite, stomach discomfort, photosensitivity, and diarrhea. It is reported that more than 30% of patients choose to reduce the dose, temporarily discontinue medication, or stop treatment (Levra S, et al. Long-Term Safety of Antifibrotic Drugs in IPF: A Real-World Experience. Biomedicines. 2022 Dec 12; 10(12): 3229).

Mirtazapine has been approved in multiple countries for the treatment of depression. Existing reports indicate that mirtazapine can alleviate pulmonary fibrosis in experimental animal models (Abdelhady R, et al. Biomed Pharmacother. 2023 May; 161: 114553). However, mirtazapine has relatively many and severe side effects, including weight gain, fatigue (15.2%), somnolence (50%), sedation, dizziness and lassitude, headache, constipation (12.7%), dry mouth (20.6%), elevated aspartate transaminase (AST), elevated alanine transaminase (ALT) (12.4%), elevated γ-GTP, and increased appetite. To date, no literature has reported that low-dose mirtazapine alone can be used to treat organ fibrosis or its related diseases, nor that the combination of mirtazapine with other drugs exerts additive or even synergistic effects in alleviating organ fibrosis or its related diseases.

Noscapine is a peripheral antitussive. WO 2009/155611 investigated the preventive effect of noscapine administration on fibrotic diseases in experimental animal models. However, to date, no literature has reported that the combination of noscapine with other drugs exerts additive or even synergistic effects in alleviating organ fibrosis or its related diseases.

Therefore, there is an unmet medical need for new, safe drugs and methods for long-term medication of patients for the treatment of organ fibrosis related diseases.

### Summary of the Invention

The objective of the present invention is to provide new drugs and methods for treating an organ fibrosis related disease in subjects in need, and preferably to provide new drugs and methods with high safety for long-term medication of patients.

In experiments, the inventors unexpectedly found that mirtazapine, when used alone or in combination with certain marketed drugs, can significantly inhibit organ fibrosis in mammals. Particularly unexpectedly, it was discovered that when used alone, among the two enantiomers of mirtazapine, only the R-configuration mirtazapine exerts a statistically significant antifibrotic effect, whereas the S-configuration mirtazapine, which is considered the main active form for antidepressant effects, has no statistically significant antifibrotic effect. Furthermore, the antifibrotic effect of mirtazapine can be achieved at a relatively low dose (i.e., lower than its effective dose for treating depression, such as a daily dose of less than 15 mg, e.g., a daily dose of 1 to 10 mg). Based on this discovery, the inventors completed the present invention, which thus achieves the aforementioned objective.

The present invention can be described from different aspects. The inventions described in any of these aspects and any of their embodiments are independent yet interrelated, collectively constituting the content of the present invention.

One aspect of the present invention provides a method for treating an organ fibrosis related disease in a patient, comprising a step of administering a therapeutically effective amount of mirtazapine or a pharmaceutically acceptable salt thereof to the patient.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, a method for treating progressive fibrosing interstitial lung disease in a patient is provided, the method comprising a step of administering a therapeutically effective amount of mirtazapine or a pharmaceutically acceptable salt thereof to the patient.

In some embodiments, a method for treating progressive pulmonary fibrosis in a patient is provided, the method comprising a step of administering a therapeutically effective amount of mirtazapine or a pharmaceutically acceptable salt thereof to the patient.

In some embodiments, a method for treating idiopathic pulmonary fibrosis in a patient is provided, the method comprising a step of administering a therapeutically effective amount of mirtazapine or a pharmaceutically acceptable salt thereof to the patient.

In some embodiments, the therapeutically effective amount of mirtazapine or a pharmaceutically acceptable salt thereof is administered to a subject in need by oral administration of a pharmaceutical composition comprising mirtazapine or a pharmaceutically acceptable salt thereof and a pharmaceutically acceptable excipient.

In some embodiments, the therapeutically effective amount of mirtazapine in a single dose is 0.1 to 45 mg, e.g., 1 to 10 mg, preferably 3 to 5 mg, administered once or multiple times a day.

In some embodiments, the mirtazapine is substantially pure R-configuration mirtazapine, i.e., substantially pure (R)-mirtazapine.

Another aspect of the present invention provides the use of mirtazapine or a pharmaceutically acceptable salt thereof in the manufacture of a medicament for treating an organ fibrosis related disease in a patient in need thereof.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, the use of mirtazapine or a pharmaceutically acceptable salt thereof in the manufacture of a medicament for treating progressive fibrosing interstitial lung disease in a patient in need thereof is provided.

In some embodiments, the use of mirtazapine or a pharmaceutically acceptable salt thereof in the manufacture of a medicament for treating progressive pulmonary fibrosis in a patient in need thereof is provided.

In some embodiments, the use of mirtazapine or a pharmaceutically acceptable salt thereof in the manufacture of a medicament for treating idiopathic pulmonary fibrosis in a patient in need thereof is provided.

In some embodiments, the mirtazapine is substantially pure R-configuration mirtazapine, i.e., substantially pure (R)-mirtazapine.

Yet another aspect of the present invention provides a pharmaceutical composition comprising mirtazapine or a pharmaceutically acceptable salt thereof and a pharmaceutically acceptable excipient, for use in treating an organ fibrosis related disease in a patient in need thereof.

In some embodiments, mirtazapine is administered in a therapeutically effective amount.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, a pharmaceutical composition comprising mirtazapine or a pharmaceutically acceptable salt thereof and a pharmaceutically acceptable excipient is provided, for use in treating progressive fibrosing interstitial lung disease in a patient in need thereof.

In some embodiments, a pharmaceutical composition comprising mirtazapine or a pharmaceutically acceptable salt thereof and a pharmaceutically acceptable excipient is provided, for use in treating progressive pulmonary fibrosis in a patient in need thereof.

In some embodiments, a pharmaceutical composition comprising mirtazapine or a pharmaceutically acceptable salt thereof and a pharmaceutically acceptable excipient is provided, for use in treating idiopathic pulmonary fibrosis in a patient in need thereof.

In some embodiments, the therapeutically effective amount of mirtazapine in a single dose is 0.1 to 45 mg, e.g., 1 to 10 mg, preferably 3 to 5 mg, administered once or multiple times a day.

In some embodiments, the mirtazapine is substantially pure R-configuration mirtazapine, i.e., substantially pure (R)-mirtazapine.

Yet another aspect of the present invention provides substantially pure (R)-mirtazapine or a pharmaceutically acceptable salt thereof, for use in a medicament for improving pulmonary fibrosis lesions, inhibiting the progression of pulmonary fibrosis, or reducing the severity of pulmonary fibrosis in a patient.

In some embodiments, mirtazapine is administered in a therapeutically effective amount for use in a medicament for improving pulmonary fibrosis lesions, inhibiting the progression of pulmonary fibrosis, or reducing the severity of pulmonary fibrosis in a patient.

In some embodiments, the therapeutically effective amount of mirtazapine in a single dose is 0.1 to 45 mg, e.g., 1 to 10 mg, preferably 3 to 5 mg, administered once or multiple times a day.

Yet another aspect of the present invention provides substantially pure (R)-mirtazapine or a pharmaceutically acceptable salt thereof, for use in treating an organ fibrosis related disease in a patient, wherein the organ fibrosis related disease may be one or more selected from fibrosing interstitial lung diseases, renal interstitial fibrosis diseases, liver fibrosis diseases, and myocardial fibrosis diseases. In some embodiments, mirtazapine is administered in a therapeutically effective amount for treating organ fibrosis related diseases.

In some embodiments, the therapeutically effective amount of mirtazapine in a single dose is 0.1 to 45 mg, e.g., 1 to 10 mg, preferably 3 to 5 mg, administered once or multiple times a day.

Yet another aspect of the present invention provides a method for treating an organ fibrosis related disease in a patient, comprising a step of administering a therapeutically effective amount of mirtazapine or a pharmaceutically acceptable salt thereof in combination with a therapeutically effective amount of noscapine or a pharmaceutically acceptable salt thereof to the patient.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, a method for treating progressive fibrosing interstitial lung disease in a patient is provided, the method comprising a step of administering a therapeutically effective amount of mirtazapine or a pharmaceutically acceptable salt thereof in combination with a therapeutically effective amount of noscapine or a pharmaceutically acceptable salt thereof to the patient.

In some embodiments, a method for treating progressive pulmonary fibrosis in a patient is provided, the method comprising a step of administering a therapeutically effective amount of mirtazapine or a pharmaceutically acceptable salt thereof in combination with a therapeutically effective amount of noscapine or a pharmaceutically acceptable salt thereof to the patient.

In some embodiments, a method for treating idiopathic pulmonary fibrosis in a patient is provided, the method comprising a step of administering a therapeutically effective amount of mirtazapine or a pharmaceutically acceptable salt thereof in combination with a therapeutically effective amount of noscapine or a pharmaceutically acceptable salt thereof to the patient.

In some embodiments, the therapeutically effective amount of mirtazapine in a single dose is 0.1 to 45 mg, e.g., 1 to 10 mg, preferably 3 to 5 mg, administered once or multiple times a day.

In some embodiments, the therapeutically effective amount of noscapine in a single dose is 1 to 3000 mg, preferably 10 to 120 mg, administered once or multiple times a day.

In some embodiments, the mirtazapine is substantially pure R-configuration mirtazapine, i.e., substantially pure (R)-mirtazapine.

Yet another aspect of the present invention provides the use of a combination of mirtazapine or a pharmaceutically acceptable salt thereof and noscapine or a pharmaceutically acceptable salt thereof in the manufacture of a medicament for treating an organ fibrosis related disease in a patient in need thereof.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, the use of a combination of mirtazapine or a pharmaceutically acceptable salt thereof and noscapine or a pharmaceutically acceptable salt thereof in the manufacture of a medicament for treating progressive fibrosing interstitial lung disease in a patient in need thereof is provided.

In some embodiments, the use of a combination of mirtazapine or a pharmaceutically acceptable salt thereof and noscapine or a pharmaceutically acceptable salt thereof in the manufacture of a medicament for treating progressive pulmonary fibrosis in a patient in need thereof is provided.

In some embodiments, the use of a combination of mirtazapine or a pharmaceutically acceptable salt thereof and noscapine or a pharmaceutically acceptable salt thereof in the manufacture of a medicament for treating idiopathic pulmonary fibrosis in a patient in need thereof is provided.

In some embodiments, mirtazapine or a pharmaceutically acceptable salt thereof and noscapine or a pharmaceutically acceptable salt thereof are formulated in the same pharmaceutical preparation.

In some embodiments, mirtazapine or a pharmaceutically acceptable salt thereof and noscapine or a pharmaceutically acceptable salt thereof are each formulated in separate pharmaceutical formulations.

In some embodiments, the mirtazapine is substantially pure R-configuration mirtazapine, i.e., substantially pure (R)-mirtazapine.

Yet another aspect of the present invention provides a pharmaceutical composition comprising mirtazapine or a pharmaceutically acceptable salt thereof, noscapine or a pharmaceutically acceptable salt thereof, and a pharmaceutically acceptable excipient.

In some embodiments, mirtazapine and noscapine are each administered in their respective therapeutically effective amounts to a patient in need thereof.

In some embodiments, the therapeutically effective amount of mirtazapine in a single dose is 0.1 to 45 mg, e.g., 1 to 10 mg, preferably 3 to 5 mg, administered once or multiple times a day.

In some practical embodiments, the therapeutically effective amount of noscapine is 1 to 3000 mg, preferably 10 to 120 mg, administered once or multiple times a day.

In some embodiments, the mirtazapine is substantially pure R-configuration mirtazapine, i.e., substantially pure (R)-mirtazapine.

Yet another aspect of the present invention provides a pharmaceutical composition comprising mirtazapine or a pharmaceutically acceptable salt thereof, noscapine or a pharmaceutically acceptable salt thereof, and a pharmaceutically acceptable excipient, wherein the pharmaceutical composition is for treating an organ fibrosis related disease in a patient in need thereof.

In some embodiments, mirtazapine and noscapine are each administered in their respective therapeutically effective amounts for treating an organ fibrosis related disease in a patient in need thereof.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, a pharmaceutical composition is provided, comprising mirtazapine or a pharmaceutically acceptable salt thereof, noscapine or a pharmaceutically acceptable salt thereof, and a pharmaceutically acceptable excipient, wherein the pharmaceutical composition is for treating progressive fibrosing interstitial lung disease in a patient in need thereof.

In some embodiments, a pharmaceutical composition is provided, comprising mirtazapine or a pharmaceutically acceptable salt thereof, noscapine or a pharmaceutically acceptable salt thereof, and a pharmaceutically acceptable excipient, wherein the pharmaceutical composition is for treating progressive pulmonary fibrosis in a patient in need thereof.

In some embodiments, a pharmaceutical composition is provided, comprising mirtazapine or a pharmaceutically acceptable salt thereof, noscapine or a pharmaceutically acceptable salt thereof, and a pharmaceutically acceptable excipient, wherein the pharmaceutical composition is for treating idiopathic pulmonary fibrosis in a patient in need thereof.

In some embodiments, the therapeutically effective amount of mirtazapine in a single dose is 0.1 to 45 mg, e.g., 1 to 10 mg, preferably 3 to 5 mg, administered once or multiple times a day.

In some practical embodiments, the therapeutically effective amount of noscapine in a single dose is 1 to 3000 mg, preferably 10 to 120 mg, administered once or multiple times a day.

In some embodiments, the mirtazapine is substantially pure R-configuration mirtazapine, i.e., substantially pure (R)-mirtazapine.

Yet another aspect of the present invention provides a pharmaceutical combination comprising a therapeutically effective amount of mirtazapine or a pharmaceutically acceptable salt thereof and a therapeutically effective amount of noscapine or a pharmaceutically acceptable salt thereof, wherein mirtazapine or a pharmaceutically acceptable salt thereof and noscapine or a pharmaceutically acceptable salt thereof may be formulated in the same pharmaceutical preparation or in separate pharmaceutical formulations.

In some embodiments, the therapeutically effective amount of mirtazapine in a single dose is 0.1 to 45 mg, e.g., 1 to 10 mg, preferably 3 to 5 mg, administered once or multiple times a day.

In some practical embodiments, the therapeutically effective amount of noscapine in a single dose is 1 to 3000 mg, preferably 10 to 120 mg, administered once or multiple times a day.

In some embodiments, the mirtazapine is substantially pure R-configuration mirtazapine, i.e., substantially pure (R)-mirtazapine.

Yet another aspect of the present invention provides a pharmaceutical kit comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat organ fibrosis related diseases in a patient in need thereof.

In some embodiments, the organ fibrosis related disease is selected from one or more of fibrosing interstitial lung diseases, renal interstitial fibrosis diseases, liver fibrosis diseases, and myocardial fibrosis diseases.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat progressive fibrosing interstitial lung disease in a patient in need thereof.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat progressive pulmonary fibrosis in a patient in need thereof.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat idiopathic pulmonary fibrosis in a patient in need thereof.

In some embodiments, the mirtazapine is substantially pure R-configuration mirtazapine, i.e., substantially pure (R)-mirtazapine.

The inventors have confirmed through experiments that mirtazapine or a pharmaceutically acceptable salt thereof can effectively inhibit pulmonary fibrosis in mammals when administered alone or in combination with noscapine or a pharmaceutically acceptable salt thereof. Particularly unexpectedly, the combination of low-dose mirtazapine or a pharmaceutically acceptable salt thereof and noscapine or a pharmaceutically acceptable salt thereof inhibits pulmonary fibrosis more significantly than existing marketed drugs.

The inventors also found in experiments that the antifibrotic effect of mirtazapine in combination with other drugs is unpredictable. For example, experiments have shown that when mirtazapine is combined with simvastatin, the antifibrotic effect of the combination is less than the sum of their individual antifibrotic effects when used alone. In contrast, in the present invention, when mirtazapine is combined with noscapine, the antifibrotic effect of the combination is greater than the sum of their individual antifibrotic effects when used alone, i.e., a synergistic effect is produced.

Therefore, it can be expected that the methods, pharmaceutical combinations, and pharmaceutical compositions of the present invention can effectively inhibit organ fibrosis in patients, thereby treating organ fibrosis related diseases, for example, fibrosing interstitial lung diseases e.g., progressive fibrosing interstitial lung disease, progressive pulmonary fibrosis, and idiopathic pulmonary fibrosis, in these patients, and alleviating symptoms such as mental stress, dyspnea, and cough in these patients. Based on the experimental results obtained by the inventors, it can also be expected that the methods, pharmaceutical combinations, and pharmaceutical compositions of the present invention can inhibit fibrotic lesions in other organs, such as renal fibrotic lesions, hepatic fibrotic lesions, and cardiac fibrotic lesions. The dose of mirtazapine as used herein is low, and the combined administration of mirtazapine and noscapine not only results in fewer and milder side effects than administration of either drug alone but also exhibits a stronger antifibrotic effect. Therefore, it can also be expected that the methods, pharmaceutical combinations, and pharmaceutical compositions of the present invention can avoid or reduce the side effects of mirtazapine while achieving an effective antifibrotic effect, and have higher safety compared with marketed antifibrotic drugs, thereby improving patient compliance.

### Detailed Description of Embodiments

In order to facilitate the understanding of the present invention, certain terms as used herein are defined here. The definitions of these terms should be understood in conjunction with the understanding of the rest of the present specification by those skilled in the art. Unless otherwise specifically defined in the present specification, all technical and scientific terms as used herein have the same meanings as those commonly understood by those skilled in the art. If the meaning of a term specifically defined in the present specification is inconsistent with the meaning commonly understood by those skilled in the art, the meaning specifically defined in the present specification shall prevail.

As used herein, the term "organ fibrosis related diseases" refers to fibrotic lesions and diseases associated or caused thereby occurring in the internal organs of mammals, especially humans, including one or more clinical symptoms of these diseases, including but not limited to diseases or symptoms caused by heart fibrosis, liver fibrosis, lung fibrosis, and kidney fibrosis respectively. Fibrosis refers to the excessive deposition of extracellular matrix proteins, scarring and thickening of tissues.

As used herein, the term "fibrosing interstitial lung diseases" refers to fibrotic lesions and diseases associated or caused thereby occurring in the lungs of mammals, especially humans. Examples of fibrosing interstitial lung diseases include fibrotic interstitial lung diseases or pulmonary fibrosis, including idiopathic pulmonary fibrosis and progressive fibrosing interstitial lung diseases.

As used herein, the term "progressive fibrosing interstitial lung diseases" refers to a group of interstitial lung diseases in which pulmonary fibrosis in patients continues to progress even after treatment with immunosuppressants and elimination of factors that promote disease progression. In some embodiments, "progressive fibrosing interstitial lung diseases" is also referred to as "progressive pulmonary fibrosis (PPF)", and they are used interchangeably herein. Progressive fibrosing interstitial lung diseases include idiopathic non-specific interstitial pneumonia (INSIP), connective tissue disease-associated interstitial lung disease (CTD-ILD), hypersensitivity pneumonitis (HP), unclassifiable interstitial lung disease (UILD), pneumoconiosis, and sarcoidosis, etc.

As used herein, the term "idiopathic pulmonary fibrosis" refers to a group of diseases characterized primarily by pulmonary fibrosis, which can lead to the progressive loss of normal lung function. Its specific manifestations include progressive dyspnea, cough, expectoration, and other related respiratory symptoms. As injury and disease severity increase, respiratory function gradually fails. The clinical incidence of such diseases is increasing year by year, and the average survival time after diagnosis is 2.5 to 3 years. The pathogenesis of pulmonary fibrosis remains unclear. Currently, it is believed that alveolar epithelial cell damage, inflammatory responses, alveolar macrophages, and cell apoptosis are all key target mechanisms in the pathogenesis of pulmonary fibrosis.

As used herein, the term "treatment" refers to the prevention, improvement, alleviation, or elimination of at least one distinguishable symptom of the aforementioned organ fibrosis related diseases, including the reduction of the degree of fibrosis in an organ or tissue. In particular, the term "treatment" as used herein refers to reducing the severity of fibrosis in the affected organ or tissue, improving the physiological function of the affected organ or tissue, and/or preventing the occurrence of varying degrees of fibrosis in the affected organ or tissue.

As used herein, the terms "subject", "recipient", and "patient" have the same meaning and are used interchangeably herein. They refer to mammals, especially humans, that already have the aforementioned fibrotic disease or are at risk of developing the aforementioned fibrotic disease.

Unless otherwise specified, the phrase "therapeutically effective amount" as used herein refers to the amount of the drug or compound that, when administered to a patient, provides the desired therapeutic effect. The desired therapeutic effect includes reducing the severity of fibrosis in the affected organ or tissue, improving the physiological function of the affected organ or tissue, and/or preventing the occurrence of varying degrees of fibrosis in the affected organ or tissue. Those skilled in the art understand that the "therapeutically effective amount" of the drugs as used herein can be determined and adjusted based on factors such as the patient's age, weight, gender, type and severity of the disease, and route of administration.

The term "mirtazapine" as used herein refers to an organic compound with the chemical structural formula 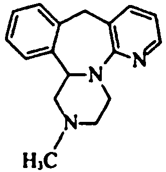 , the chemical name 1,2,3,4,10,14b-hexahydro-2-methylpyrazino[2,1-a]pyrido[2,3-c][2]benzazepine, and the molecular formula C₁₇H₁₉N₃. For example, this compound is disclosed in U.S. Patent No. 4,062,848, the entire contents of which are incorporated herein by reference. This compound contains a chiral center and can exist in the form of different enantiomers (i.e., R-configuration mirtazapine and S-configuration mirtazapine) and mixtures of these enantiomers. In embodiments where mirtazapine or a pharmaceutically acceptable salt thereof is used alone, "mirtazapine" refers to a mixture of both R-configuration mirtazapine and S-configuration mirtazapine, e.g., racemic mirtazapine, preferably substantially pure (R)-mirtazapine. In embodiments where mirtazapine or a pharmaceutically acceptable salt thereof is used in combination with noscapine or a pharmaceutically acceptable salt thereof, "mirtazapine" refers to R-configuration mirtazapine, S-configuration mirtazapine, or a mixture of R-configuration mirtazapine and S-configuration mirtazapine in any ratio.

The term "substantially pure (R)-mirtazapine" as used herein refers to a mixture of (R)-mirtazapine and (S)-mirtazapine, wherein the content of (R)-mirtazapine is greater than 50%, 60%, 70%, 80%, 90%, 95%, 96%, 97%, 98%, 99%, or 99.5%.

The pharmaceutically acceptable salts of mirtazapine as used herein include various pharmaceutically acceptable salts of mirtazapine, such as hydrochloride, hydrobromide, hydroiodide, nitrate, sulfate, bisulfate, phosphate, formate, acetate, lactate, citrate, tartrate, ascorbate, succinate, maleate, fumarate, gluconate, glucuronate, benzoate, glutamate, methanesulfonate, ethanesulfonate, benzenesulfonate, p-toluenesulfonate, and pamoate.

Mirtazapine as described herein also includes all metabolites and analogs known in the art that possess the same pharmacological activity as mirtazapine. Mirtazapine or a pharmaceutically acceptable salt thereof as used herein includes solvates (e.g., hydrates, etc.). A solvate can coordinate with any number of solvent molecules (e.g., water molecules, etc.) to form mirtazapine or a pharmaceutically acceptable salt thereof. Exposing mirtazapine or a pharmaceutically acceptable salt thereof to the atmosphere may cause it to absorb moisture, and the adsorbed water may adhere to it or form a hydrate. The preparation of mirtazapine, its pharmaceutically acceptable salts and solvates (e.g., hydrates, etc.) is known to those skilled in the art.

Mirtazapine as used herein also includes its isotopically labeled forms. Isotopically labeled mirtazapine is structurally identical to the compound shown in the above formula except that one or more atoms are replaced by atoms having an atomic weight or mass number different from those typically found in nature. Examples of applicable isotopes include isotopes of hydrogen, carbon, and nitrogen, such as but not limited to ²H, ³H, ¹³C, ¹⁴C, and ¹⁵N. In general, isotopically labeled mirtazapine can be prepared using methods known in the art by replacing non-isotopically labeled reagents with readily available isotopically labeled reagents.

The term "noscapine" as used herein refers to a peripheral antitussive, with the chemical name [S-(R*,S*)]-6,7-dimethoxy-3-(5,6,7,8-tetrahydro-6-methyl-4-methoxy-1,3-dioxoleno[4,5-g]-5-isoquinolinyl)-1(3H)-isobenzofuranone, the molecular formula C₂₂H₂₃NO₇ and the chemical structural formula 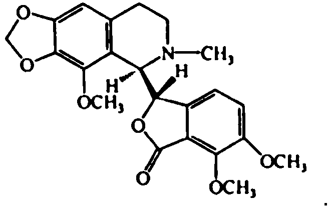 Noscapine as described herein also includes all metabolites and analogs known in the art that possess the same pharmacological activity as noscapine. The pharmaceutically acceptable salts of noscapine as used herein include various pharmaceutically acceptable salts of noscapine, such as hydrochloride, hydrobromide, hydroiodide, nitrate, sulfate, bisulfate, phosphate, formate, acetate, lactate, citrate, tartrate, ascorbate, succinate, maleate, fumarate, gluconate, glucuronate, benzoate, glutamate, methanesulfonate, ethanesulfonate, benzenesulfonate, p-toluenesulfonate, and pamoate.

Noscapine or a pharmaceutically acceptable salt thereof as used herein includes solvates (e.g., hydrates, etc.). A solvate can coordinate with any number of solvent molecules (e.g., water molecules, etc.) to form noscapine or a pharmaceutically acceptable salt thereof. Exposing noscapine or a pharmaceutically acceptable salt thereof to the atmosphere may cause it to absorb moisture, and the adsorbed water may adhere to it or form a hydrate. The preparation of noscapine, its pharmaceutically acceptable salts and solvates (e.g., hydrates, etc.) is known to those skilled in the art.

Noscapine as used herein also includes its isotopically labeled forms. Isotopically labeled noscapine is structurally identical to the compound shown in the above formula except that one or more atoms are replaced by atoms having an atomic weight or mass number different from those typically found in nature. Examples of applicable isotopes include isotopes of hydrogen, carbon, nitrogen, and oxygen, such as but not limited to ²H, ³H, ¹³C, ¹⁴C, ¹⁵N, ¹⁷O and ¹⁸O. In general, isotopically labeled noscapine can be prepared using methods known in the art by replacing non-isotopically labeled reagents with readily available isotopically labeled reagents.

As used herein, the expressions "administering mirtazapine or a pharmaceutically acceptable salt thereof and noscapine or a pharmaceutically acceptable salt thereof simultaneously" and "administering mirtazapine or a pharmaceutically acceptable salt thereof in combination with noscapine or a pharmaceutically acceptable salt thereof" have the same meaning and are used interchangeably in the present invention. The expression "administering mirtazapine or a pharmaceutically acceptable salt thereof in combination with noscapine or a pharmaceutically acceptable salt thereof" as used herein refers to administering mirtazapine or a pharmaceutically acceptable salt thereof to a subject in need before, after, or substantially at the same time as administering noscapine or a pharmaceutically acceptable salt thereof.

According to one aspect of the present invention, a method for treating an organ fibrosis related disease in a patient is provided, the method comprising a step of administering a therapeutically effective amount of mirtazapine or a pharmaceutically acceptable salt thereof to the patient, wherein the organ fibrosis related disease is selected from one or more of fibrosing interstitial lung diseases, renal interstitial fibrosis diseases, liver fibrosis diseases, and myocardial fibrosis diseases.

In some embodiments, a method for treating renal interstitial fibrosis disease in a patient is provided, the method comprising administering a therapeutically effective amount of mirtazapine or a pharmaceutically acceptable salt thereof to the patient.

In some embodiments, a method for inhibiting renal interstitial fibrosis or reducing the degree of renal interstitial fibrosis in a patient is provided, the method comprising administering a therapeutically effective amount of mirtazapine or a pharmaceutically acceptable salt thereof to the patient.

In some embodiments, a method for treating liver fibrosis disease in a patient is provided, the method comprising administering a therapeutically effective amount of mirtazapine or a pharmaceutically acceptable salt thereof to the patient.

In some embodiments, a method for inhibiting hepatic fibrosis or reducing the degree of hepatic fibrosis in a patient is provided, the method comprising administering a therapeutically effective amount of mirtazapine or a pharmaceutically acceptable salt thereof to the patient.

In some embodiments, a method for treating myocardial fibrosis disease in a patient is provided, the method comprising administering a therapeutically effective amount of mirtazapine or a pharmaceutically acceptable salt thereof to the patient.

In some embodiments, a method for inhibiting myocardial fibrosis or reducing the degree of myocardial fibrosis in a patient is provided, the method comprising administering a therapeutically effective amount of mirtazapine or a pharmaceutically acceptable salt thereof to the patient.

In some embodiments, a method for treating progressive fibrosing interstitial lung disease in a patient is provided, the method comprising a step of administering a therapeutically effective amount of mirtazapine or a pharmaceutically acceptable salt thereof to the patient.

In some embodiments, a method for treating progressive pulmonary fibrosis in a patient is provided, the method comprising a step of administering a therapeutically effective amount of mirtazapine or a pharmaceutically acceptable salt thereof to the patient.

In some embodiments, a method for treating idiopathic pulmonary fibrosis in a patient is provided, the method comprising a step of administering a therapeutically effective amount of mirtazapine or a pharmaceutically acceptable salt thereof to the patient.

In some embodiments, a method for improving pulmonary fibrosis lesions, inhibiting the progression of pulmonary fibrosis, or reducing the degree of pulmonary fibrosis in a patient is provided, the method comprising a step of administering a therapeutically effective amount of mirtazapine or a pharmaceutically acceptable salt thereof to a subject in need.

In some embodiments, a method for treating idiopathic non-specific interstitial pneumonia in a patient is provided, the method comprising administering a therapeutically effective amount of mirtazapine or a pharmaceutically acceptable salt thereof to the patient.

In some embodiments, a method for treating connective tissue disease-associated interstitial lung disease in a patient is provided, the method comprising administering a therapeutically effective amount of mirtazapine or a pharmaceutically acceptable salt thereof to the patient.

In some embodiments, a method for treating pneumoconiosis, reducing its severity, or preventing its progression in a patient is provided, the method comprising administering a therapeutically effective amount of mirtazapine or a pharmaceutically acceptable salt thereof to the patient.

In some embodiments, a method for treating pulmonary sarcoidosis in a patient is provided, the method comprising administering a therapeutically effective amount of mirtazapine or a pharmaceutically acceptable salt thereof to the patient.

In some embodiments, a therapeutically effective amount of mirtazapine or a pharmaceutically acceptable salt thereof is administered to a subject in need by the administration of a pharmaceutical composition in the form of an oral solid preparation, wherein the pharmaceutical composition comprises mirtazapine or a pharmaceutically acceptable salt thereof and a pharmaceutically acceptable excipient.

In some embodiments, a therapeutically effective amount of mirtazapine or a pharmaceutically acceptable salt thereof is administered to a subject in need by the administration of a pharmaceutical composition in the form of a tablet, wherein the pharmaceutical composition comprises mirtazapine or a pharmaceutically acceptable salt thereof and a pharmaceutically acceptable excipient.

In some embodiments, a therapeutically effective amount of mirtazapine or a pharmaceutically acceptable salt thereof is administered to a subject in need by the administration of a pharmaceutical composition in the form of a capsule, wherein the pharmaceutical composition comprises mirtazapine or a pharmaceutically acceptable salt thereof and a pharmaceutically acceptable excipient.

In some embodiments, the treatment comprises administering mirtazapine at a dose in the range of 0.1 to 45 mg, 0.5 to 30 mg, 0.5 to 15 mg, 0.5 to 10 mg, 1 to 10 mg, or 3 to 5 mg, or a corresponding amount of a pharmaceutically acceptable salt of mirtazapine, to a subject in need once or multiple times a day.

In some embodiments, the pharmaceutical composition comprising mirtazapine or a pharmaceutically acceptable salt thereof is administered to the patient once or multiple times a day.

In some embodiments, the method comprises administering the pharmaceutical composition comprising mirtazapine or a pharmaceutically acceptable salt thereof to the patient once a day.

In some embodiments, the method comprises administering the pharmaceutical composition comprising mirtazapine or a pharmaceutically acceptable salt thereof to the patient twice a day.

In some embodiments, the pharmaceutical composition is administered to a patient in need thereof in the form of dosage units, and each dosage unit contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the mirtazapine is substantially pure R-configuration mirtazapine, i.e., substantially pure (R)-mirtazapine.

According to another aspect of the present invention, the use of mirtazapine or a pharmaceutically acceptable salt thereof in the manufacture of a medicament for treating an organ fibrosis related disease in a patient in need thereof is provided, wherein the organ fibrosis related disease is selected from one or more of fibrosing interstitial lung diseases, renal interstitial fibrosis diseases, liver fibrosis diseases, and myocardial fibrosis diseases.

In some embodiments, the use of mirtazapine or a pharmaceutically acceptable salt thereof in the manufacture of a medicament for treating renal interstitial fibrosis disease in a patient in need thereof is provided.

In some embodiments, the use of mirtazapine or a pharmaceutically acceptable salt thereof in the manufacture of a medicament for inhibiting renal interstitial fibrosis or reducing the degree of renal interstitial fibrosis is provided.

In some embodiments, the use of mirtazapine or a pharmaceutically acceptable salt thereof in the manufacture of a medicament for treating liver fibrosis disease in a patient in need thereof is provided.

In some embodiments, the use of mirtazapine or a pharmaceutically acceptable salt thereof in the manufacture of a medicament for inhibiting hepatic fibrosis or reducing the degree of hepatic fibrosis is provided.

In some embodiments, the use of mirtazapine or a pharmaceutically acceptable salt thereof in the manufacture of a medicament for treating myocardial fibrosis disease in a patient in need thereof is provided.

In some embodiments, the use of mirtazapine or a pharmaceutically acceptable salt thereof in the manufacture of a medicament for inhibiting myocardial fibrosis or reducing the degree of myocardial fibrosis is provided.

In some embodiments, the use of mirtazapine or a pharmaceutically acceptable salt thereof in the manufacture of a medicament for treating idiopathic non-specific interstitial pneumonia in a patient in need thereof is provided.

In some embodiments, the use of mirtazapine or a pharmaceutically acceptable salt thereof in the manufacture of a medicament for treating connective tissue disease-associated interstitial lung disease in a patient in need thereof is provided.

In some embodiments, the use of mirtazapine or a pharmaceutically acceptable salt thereof in the manufacture of a medicament for treating pneumoconiosis, reducing its severity, or preventing its progression in a patient in need thereof is provided.

In some embodiments, the use of mirtazapine or a pharmaceutically acceptable salt thereof in the manufacture of a medicament for treating pulmonary sarcoidosis in a patient in need thereof is provided.

In some embodiments, the use of mirtazapine or a pharmaceutically acceptable salt thereof in the manufacture of a medicament for improving pulmonary fibrosis lesions, inhibiting the progression of pulmonary fibrosis, or reducing the degree of pulmonary fibrosis in a patient is provided.

In some embodiments, the use of mirtazapine or a pharmaceutically acceptable salt thereof in the manufacture of a medicament for treating progressive fibrosing interstitial lung disease in a patient in need thereof is provided.

In some embodiments, the use of mirtazapine or a pharmaceutically acceptable salt thereof in the manufacture of a medicament for treating progressive pulmonary fibrosis in a patient in need thereof is provided.

In some embodiments, the use of mirtazapine or a pharmaceutically acceptable salt thereof in the manufacture of a medicament for treating idiopathic pulmonary fibrosis in a patient in need thereof is provided.

In some embodiments, the medicament is prepared in the form of dosage units, such as tablets or capsules, which contain mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the use of mirtazapine or a pharmaceutically acceptable salt thereof in the manufacture of a medicament for treating progressive fibrosing interstitial lung disease, progressive pulmonary fibrosis, or idiopathic pulmonary fibrosis in a patient in need thereof is provided, wherein the medicament contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the mirtazapine is substantially pure R-configuration mirtazapine, i.e., substantially pure (R)-mirtazapine.

According to yet another aspect of the present invention, a pharmaceutical composition comprising mirtazapine or a pharmaceutically acceptable salt thereof and a pharmaceutically acceptable excipient is provided, for use in treating an organ fibrosis related disease in a patient in need thereof, wherein the organ fibrosis related disease is selected from one or more of fibrosing interstitial lung diseases, renal interstitial fibrosis diseases, liver fibrosis diseases, and myocardial fibrosis diseases.

In some embodiments, a pharmaceutical composition comprising mirtazapine or a pharmaceutically acceptable salt thereof and a pharmaceutically acceptable excipient is provided, for use in treating renal interstitial fibrosis in a patient in need thereof.

In some embodiments, a pharmaceutical composition comprising mirtazapine or a pharmaceutically acceptable salt thereof and a pharmaceutically acceptable excipient is provided, for use in inhibiting renal interstitial fibrosis or reducing the degree of renal interstitial fibrosis.

In some embodiments, a pharmaceutical composition comprising mirtazapine or a pharmaceutically acceptable salt thereof and a pharmaceutically acceptable excipient is provided, for use in treating liver fibrosis in a patient in need thereof.

In some embodiments, a pharmaceutical composition comprising mirtazapine or a pharmaceutically acceptable salt thereof and a pharmaceutically acceptable excipient is provided, for use in inhibiting hepatic fibrosis or reducing the degree of hepatic fibrosis.

In some embodiments, a pharmaceutical composition comprising mirtazapine or a pharmaceutically acceptable salt thereof and a pharmaceutically acceptable excipient is provided, for use in treating myocardial fibrosis in a patient in need thereof.

In some embodiments, a pharmaceutical composition comprising mirtazapine or a pharmaceutically acceptable salt thereof and a pharmaceutically acceptable excipient is provided, for use in inhibiting myocardial fibrosis or reducing the degree of myocardial fibrosis.

In some embodiments, a pharmaceutical composition comprising mirtazapine or a pharmaceutically acceptable salt thereof and a pharmaceutically acceptable excipient is provided, for use in treating progressive fibrosing interstitial lung disease in a patient in need thereof.

In some embodiments, a pharmaceutical composition comprising mirtazapine or a pharmaceutically acceptable salt thereof and a pharmaceutically acceptable excipient is provided, for use in treating progressive pulmonary fibrosis in a patient in need thereof.

In some embodiments, a pharmaceutical composition comprising mirtazapine or a pharmaceutically acceptable salt thereof and a pharmaceutically acceptable excipient is provided, for use in treating idiopathic pulmonary fibrosis in a patient in need thereof.

In some embodiments, a pharmaceutical composition comprising mirtazapine or a pharmaceutically acceptable salt thereof and a pharmaceutically acceptable excipient is provided, for use in improving pulmonary fibrosis lesions, inhibiting the progression of pulmonary fibrosis, or reducing the degree of pulmonary fibrosis in a patient.

In some embodiments, a pharmaceutical composition comprising mirtazapine or a pharmaceutically acceptable salt thereof and a pharmaceutically acceptable excipient is provided, for use in treating idiopathic non-specific interstitial pneumonia in a patient in need thereof.

In some embodiments, a pharmaceutical composition comprising mirtazapine or a pharmaceutically acceptable salt thereof and a pharmaceutically acceptable excipient is provided, for use in treating connective tissue disease-associated interstitial lung disease in a patient in need thereof.

In some embodiments, a pharmaceutical composition comprising mirtazapine or a pharmaceutically acceptable salt thereof and a pharmaceutically acceptable excipient is provided, for use in treating pneumoconiosis, reducing its severity, or preventing its progression in a patient in need thereof.

In some embodiments, a pharmaceutical composition comprising mirtazapine or a pharmaceutically acceptable salt thereof and a pharmaceutically acceptable excipient is provided, for use in treating pulmonary sarcoidosis in a patient in need thereof.

In some embodiments, the treatment comprises administering mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine, to a subject in need once or multiple times a day.

In some embodiments, the pharmaceutical composition is in the form of an oral solid preparation, such as a tablet or a capsule.

In some embodiments, the pharmaceutical composition comprises mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the mirtazapine is substantially pure R-configuration mirtazapine, i.e., substantially pure (R)-mirtazapine.

According to yet another aspect of the present invention, a method for treating an organ fibrosis related disease in a patient is provided, the method comprising a step of administering to the patient a therapeutically effective amount of mirtazapine or a pharmaceutically acceptable salt thereof in combination with a therapeutically effective amount of noscapine or a pharmaceutically acceptable salt thereof, wherein the organ fibrosis related disease is selected from one or more of fibrosing interstitial lung diseases, renal interstitial fibrosis diseases, liver fibrosis diseases, and myocardial fibrosis diseases.

In some embodiments, a method for treating progressive fibrosing interstitial lung disease in a patient is provided, the method comprising a step of administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating progressive pulmonary fibrosis in a patient is provided, the method comprising a step of administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating idiopathic pulmonary fibrosis in a patient is provided, the method comprising a step of administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating renal interstitial fibrosis disease in a patient is provided, the method comprising a step of administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for inhibiting renal interstitial fibrosis or reducing the degree of renal interstitial fibrosis in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating liver fibrosis disease in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for inhibiting hepatic fibrosis or reducing the degree of hepatic fibrosis in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating myocardial fibrosis disease in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for inhibiting myocardial fibrosis or reducing the degree of myocardial fibrosis in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating progressive fibrosing interstitial lung disease in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating progressive pulmonary fibrosis in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating idiopathic pulmonary fibrosis in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for improving pulmonary fibrosis lesions, inhibiting the progression of pulmonary fibrosis, or reducing the degree of pulmonary fibrosis in a patient is provided, the method comprising administering to a subject in need a therapeutically effective amount of mirtazapine or a pharmaceutically acceptable salt thereof in combination with a therapeutically effective amount of noscapine or a pharmaceutically acceptable salt thereof.

In some embodiments, a method for treating idiopathic non-specific interstitial pneumonia in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating connective tissue disease-associated interstitial lung disease in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating pneumoconiosis, reducing its severity, or preventing its progression in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, a method for treating pulmonary sarcoidosis in a patient is provided, the method comprising administering to the patient a therapeutically effective amount of mirtazapine or a pharmaceutically acceptable salt thereof in combination with a therapeutically effective amount of noscapine or a pharmaceutically acceptable salt thereof.

In some embodiments, the therapeutically effective amount of mirtazapine or a pharmaceutically acceptable salt thereof in combination with a therapeutically effective amount of noscapine or a pharmaceutically acceptable salt thereof is administered to a subject in need by the administration of a pharmaceutical composition in the form of an oral solid preparation, wherein the pharmaceutical composition may be a single preparation comprising noscapine or a pharmaceutically acceptable salt thereof, mirtazapine or a pharmaceutically acceptable salt thereof, and a pharmaceutically acceptable excipient; alternatively, it may be separate preparations, each containing either noscapine or a pharmaceutically acceptable salt thereof, or mirtazapine or a pharmaceutically acceptable salt thereof, and a pharmaceutically acceptable excipient.

In some embodiments, the oral solid preparation is in the form of a tablet or capsule.

In some embodiments, the therapeutically effective amount of noscapine in a single dose is 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, administered once or multiple times a day, preferably once a day. Based on the therapeutically effective amount of noscapine, those skilled in the art can easily determine the therapeutically effective amount of a pharmaceutically acceptable salt of noscapine.

In some embodiments, the therapeutically effective amount of mirtazapine in a single dose is 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg. Based on the therapeutically effective amount of mirtazapine, those skilled in the art can easily determine the therapeutically effective amount of a pharmaceutically acceptable salt of mirtazapine.

In some embodiments, the pharmaceutical composition comprising mirtazapine or a pharmaceutically acceptable salt thereof is administered to the patient once or multiple times a day.

In some embodiments, the pharmaceutical composition comprising noscapine or a pharmaceutically acceptable salt thereof is administered to the patient once or multiple times a day.

In some embodiments, the method comprises administering the pharmaceutical composition comprising mirtazapine or a pharmaceutically acceptable salt thereof to the patient once a day.

In some embodiments, the method comprises administering the pharmaceutical composition comprising mirtazapine or a pharmaceutically acceptable salt thereof to the patient twice a day.

In some embodiments, the method comprises administering the pharmaceutical composition comprising noscapine or a pharmaceutically acceptable salt thereof to the patient once a day.

In some embodiments, the method comprises administering the pharmaceutical composition comprising noscapine or a pharmaceutically acceptable salt thereof to the patient twice a day.

In some embodiments, the method comprises administering the pharmaceutical composition comprising noscapine or a pharmaceutically acceptable salt thereof to the patient three times a day.

In some embodiments, the ratio of the therapeutically effective amount of mirtazapine to noscapine is from 1:30000 to 45:1, e.g., 1:120 to 10:1, preferably 1:120 to 3:1, more preferably 1:120 to 3:2, such as 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:110, 1:120, 40:1, 30:1, 20:1, 10:1, 9:1, 8:1, 7:1, 6:1, 5:1, 4:1, 3:1 or 2:1.

In some embodiments, the mirtazapine is substantially pure R-configuration mirtazapine, i.e., substantially pure (R)-mirtazapine.

According to yet another aspect of the present invention, the use of a combination of mirtazapine or a pharmaceutically acceptable salt thereof and noscapine or a pharmaceutically acceptable salt thereof in the manufacture of a medicament for treating an organ fibrosis related disease in a patient in need thereof is provided, wherein the organ fibrosis related disease is selected from one or more of fibrosing interstitial lung diseases, renal interstitial fibrosis diseases, liver fibrosis diseases, and myocardial fibrosis diseases.

In some embodiments, the use of a combination of mirtazapine or a pharmaceutically acceptable salt thereof and noscapine or a pharmaceutically acceptable salt thereof in the manufacture of a medicament for treating progressive fibrosing interstitial lung disease in a patient in need thereof is provided.

In some embodiments, the use of a combination of mirtazapine or a pharmaceutically acceptable salt thereof and noscapine or a pharmaceutically acceptable salt thereof in the manufacture of a medicament for treating progressive pulmonary fibrosis in a patient in need thereof is provided.

In some embodiments, the use of a combination of mirtazapine or a pharmaceutically acceptable salt thereof and noscapine or a pharmaceutically acceptable salt thereof in the manufacture of a medicament for treating idiopathic pulmonary fibrosis in a patient in need thereof is provided.

In some embodiments, the use of a combination of mirtazapine or a pharmaceutically acceptable salt thereof and noscapine or a pharmaceutically acceptable salt thereof in the manufacture of a medicament for improving pulmonary fibrosis lesions, inhibiting the progression of pulmonary fibrosis, or reducing the degree of pulmonary fibrosis in a patient is provided.

In some embodiments, the use of a combination of mirtazapine or a pharmaceutically acceptable salt thereof and noscapine or a pharmaceutically acceptable salt thereof in the manufacture of a medicament for treating renal interstitial fibrosis disease in a patient in need thereof is provided.

In some embodiments, the use of a combination of mirtazapine or a pharmaceutically acceptable salt thereof and noscapine or a pharmaceutically acceptable salt thereof in the manufacture of a medicament for inhibiting renal interstitial fibrosis or reducing the degree of renal interstitial fibrosis is provided.

In some embodiments, the use of a combination of mirtazapine or a pharmaceutically acceptable salt thereof and noscapine or a pharmaceutically acceptable salt thereof in the manufacture of a medicament for treating liver fibrosis disease in a patient in need thereof is provided.

In some embodiments, the use of a combination of mirtazapine or a pharmaceutically acceptable salt thereof and noscapine or a pharmaceutically acceptable salt thereof in the manufacture of a medicament for inhibiting hepatic fibrosis or reducing the degree of hepatic fibrosis is provided.

In some embodiments, the use of a combination of mirtazapine or a pharmaceutically acceptable salt thereof and noscapine or a pharmaceutically acceptable salt thereof in the manufacture of a medicament for treating myocardial fibrosis disease in a patient in need thereof is provided.

In some embodiments, the use of a combination of mirtazapine or a pharmaceutically acceptable salt thereof and noscapine or a pharmaceutically acceptable salt thereof in the manufacture of a medicament for inhibiting myocardial fibrosis or reducing the degree of myocardial fibrosis is provided.

In some embodiments, the use of a combination of mirtazapine or a pharmaceutically acceptable salt thereof and noscapine or a pharmaceutically acceptable salt thereof in the manufacture of a medicament for treating idiopathic non-specific interstitial pneumonia in a patient in need thereof is provided.

In some embodiments, the use of a combination of mirtazapine or a pharmaceutically acceptable salt thereof and noscapine or a pharmaceutically acceptable salt thereof in the manufacture of a medicament for treating connective tissue disease-associated interstitial lung disease in a patient in need thereof is provided.

In some embodiments, the use of a combination of mirtazapine or a pharmaceutically acceptable salt thereof and noscapine or a pharmaceutically acceptable salt thereof in the manufacture of a medicament for treating pneumoconiosis, reducing its severity, or preventing its progression in a patient in need thereof is provided.

In some embodiments, the use of a combination of mirtazapine or a pharmaceutically acceptable salt thereof and noscapine or a pharmaceutically acceptable salt thereof in the manufacture of a medicament for treating pulmonary sarcoidosis in a patient in need thereof is provided.

In some embodiments, the medicament is prepared as a single preparation comprising noscapine or a pharmaceutically acceptable salt thereof, mirtazapine or a pharmaceutically acceptable salt thereof, and a pharmaceutically acceptable excipient.

In some embodiments, the medicament is prepared as separate preparations, each containing either noscapine or a pharmaceutically acceptable salt thereof, or mirtazapine or a pharmaceutically acceptable salt thereof, and a pharmaceutically acceptable excipient.

In some embodiments, the medicament is prepared in the form of dosage units, e.g., tablets or capsules, where each dosage unit contains noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, e.g., 10 mg, 20 mg, 30 mg, 40 mg, 50 mg, 60 mg, 70 mg, 80 mg, 90 mg, 100 mg, 110 mg, or 120 mg, or a corresponding amount of a pharmaceutically acceptable salt of noscapine.

In some embodiments, the medicament is prepared in the form of dosage units, e.g., tablets or capsules, where each dosage unit contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the medicament is prepared in the form of dosage units, e.g., tablets or capsules, where each dosage unit contains noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, e.g., 10 mg, 20 mg, 30 mg, 40 mg, 50 mg, 60 mg, 70 mg, 80 mg, 90 mg, 100 mg, 110 mg, or 120 mg, or a corresponding amount of a pharmaceutically acceptable salt of noscapine, and mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the pharmaceutically acceptable salt of noscapine is noscapine hydrochloride.

In some embodiments, the content ratio of mirtazapine to noscapine in the manufactured medicament is from 1:30000 to 45:1, e.g., 1:120 to 10:1, preferably 1:120 to 3:1, more preferably 1:120 to 3:2, such as 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:110, 1:120, 40:1, 30:1, 20:1, 10:1, 9:1, 8:1, 7:1, 6:1, 5:1, 4:1, 3:1, or 2:1.

In some embodiments, the mirtazapine is substantially pure R-configuration mirtazapine, i.e., substantially pure (R)-mirtazapine.

According to yet another aspect of the present invention, a pharmaceutical composition is provided, comprising mirtazapine or a pharmaceutically acceptable salt thereof, noscapine or a pharmaceutically acceptable salt thereof, and a pharmaceutically acceptable excipient.

In some embodiments, the pharmaceutical composition is in the form of dosage units, e.g., tablets or capsules, where each dosage unit contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the pharmaceutical composition is in the form of dosage units, where each dosage unit contains noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, or a corresponding amount of a pharmaceutically acceptable salt thereof. For example, the pharmaceutical composition may be in the form of tablets or capsules, where each tablet or capsule contains noscapine or a pharmaceutically acceptable salt thereof at a dose in the range of 10 to 120 mg, e.g., 10 mg, 20 mg, 30 mg, 40 mg, 50 mg, 60 mg, 70 mg, 80 mg, 90 mg, 100 mg, 110 mg, or 120 mg.

In some embodiments, the pharmaceutical composition is in the form of dosage units, e.g., tablets or capsules, where each dosage unit contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine, and noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, e.g., 10 mg, 20 mg, 30 mg, 40 mg, 50 mg, 60 mg, 70 mg, 80 mg, 90 mg, 100 mg, 110 mg, or 120 mg, or a corresponding amount of a pharmaceutically acceptable salt of noscapine.

In some embodiments, the pharmaceutically acceptable salt of noscapine is noscapine hydrochloride.

In some embodiments, the content ratio of mirtazapine to noscapine in the pharmaceutical composition is from 1:30000 to 45:1, e.g., 1:120 to 10:1, preferably 1:120 to 3:1, more preferably 1:120 to 3:2, such as 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:110, 1:120, 40:1, 30:1, 20:1, 10:1, 9:1, 8:1, 7:1, 6:1, 5:1, 4:1, 3:1 or 2:1.

In some embodiments, the mirtazapine is substantially pure R-configuration mirtazapine, i.e., substantially pure (R)-mirtazapine.

According to yet another aspect of the present invention, a pharmaceutical composition is provided, comprising mirtazapine or a pharmaceutically acceptable salt thereof, noscapine or a pharmaceutically acceptable salt thereof, and a pharmaceutically acceptable excipient, wherein the pharmaceutical composition is for treating an organ fibrosis related disease in a patient in need thereof, where the organ fibrosis related disease is selected from one or more of fibrosing interstitial lung diseases, renal interstitial fibrosis diseases, liver fibrosis diseases, and myocardial fibrosis diseases.

In some embodiments, the pharmaceutical composition is used for treating progressive fibrosing interstitial lung disease in a patient.

In some embodiments, the pharmaceutical composition is used for treating progressive pulmonary fibrosis in a patient.

In some embodiments, the pharmaceutical composition is used for treating idiopathic pulmonary fibrosis in a patient in need thereof.

In some embodiments, the pharmaceutical composition is used for treating renal interstitial fibrosis disease in a patient in need thereof.

In some embodiments, the pharmaceutical composition is used for inhibiting renal interstitial fibrosis or reducing the degree of renal interstitial fibrosis in a patient in need thereof.

In some embodiments, the pharmaceutical composition is used for treating liver fibrosis disease in a patient in need thereof.

In some embodiments, the pharmaceutical composition is used for inhibiting hepatic fibrosis or reducing the degree of hepatic fibrosis in a patient in need thereof.

In some embodiments, the pharmaceutical composition is used for treating myocardial fibrosis disease in a patient in need thereof.

In some embodiments, the pharmaceutical composition is used for inhibiting myocardial fibrosis or reducing the degree of myocardial fibrosis in a patient in need thereof.

In some embodiments, the pharmaceutical composition is used for treating idiopathic non-specific interstitial pneumonia in a patient in need thereof.

In some embodiments, the pharmaceutical composition is used for treating connective tissue disease-associated interstitial lung disease in a patient in need thereof.

In some embodiments, the pharmaceutical composition is used for treating pneumoconiosis, reducing its severity, or preventing its progression in a patient in need thereof.

In some embodiments, the pharmaceutical composition is used for treating pulmonary sarcoidosis in a patient in need thereof.

In some embodiments, the pharmaceutical composition is in the form of dosage units, e.g., tablets or capsules, where each dosage unit contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine.

In some embodiments, the pharmaceutical composition is in the form of dosage units, e.g., tablets or capsules, where each dosage unit contains noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, e.g., 10 mg, 20 mg, 30 mg, 40 mg, 50 mg, 60 mg, 70 mg, 80 mg, 90 mg, 100 mg, 110 mg, or 120 mg, or a corresponding amount of a pharmaceutically acceptable salt of noscapine.

In some embodiments, the pharmaceutical composition is in the form of dosage units, e.g., tablets or capsules, where each dosage unit contains mirtazapine at a dose in the range of 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, or a corresponding amount of a pharmaceutically acceptable salt of mirtazapine, and noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, e.g., 10 mg, 20 mg, 30 mg, 40 mg, 50 mg, 60 mg, 70 mg, 80 mg, 90 mg, 100 mg, 110 mg, or 120 mg, or a corresponding amount of a pharmaceutically acceptable salt of noscapine.

In some embodiments, the pharmaceutically acceptable salt of noscapine is noscapine hydrochloride.

In some embodiments, the content ratio of mirtazapine to noscapine in the pharmaceutical composition is from 1:30000 to 45:1, e.g., 1:120 to 10:1, preferably 1:120 to 3:1, more preferably 1:120 to 3:2, such as 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:110, 1:120, 40:1, 30:1, 20:1, 10:1, 9:1, 8:1, 7:1, 6:1, 5:1, 4:1, 3:1, or 2:1.

In some embodiments, the mirtazapine is substantially pure R-configuration mirtazapine, i.e., substantially pure (R)-mirtazapine.

Yet another aspect of the present invention provides a pharmaceutical combination, comprising a therapeutically effective amount of mirtazapine or a pharmaceutically acceptable salt thereof and a therapeutically effective amount of noscapine or a pharmaceutically acceptable salt thereof.

In some embodiments, mirtazapine or a pharmaceutically acceptable salt thereof and noscapine or a pharmaceutically acceptable salt thereof are formulated into separate pharmaceutical formulations respectively, but are administered simultaneously or sequentially to a patient in need thereof for treating an organ fibrosis related disease in the patient, wherein the organ fibrosis related disease may be one or more selected from fibrosing interstitial lung diseases, renal interstitial fibrosis diseases, liver fibrosis diseases, and myocardial fibrosis diseases.

In some embodiments, the pharmaceutically acceptable salt of noscapine is noscapine hydrochloride.

In some embodiments, the ratio of the therapeutically effective amount of mirtazapine to noscapine is from 1:30000 to 45:1, e.g., 1:120 to 10:1, preferably 1:120 to 3:1, more preferably 1:120 to 3:2, such as 1:1, 1:2, 1:3, 1:4, 1:5, 1:6, 1:7, 1:8, 1:9, 1:10, 1:20, 1:30, 1:40, 1:50, 1:60, 1:70, 1:80, 1:90, 1:100, 1:110, 1:120, 40:1, 30:1, 20:1, 10:1, 9:1, 8:1, 7:1, 6:1, 5:1, 4:1, 3:1, or 2:1. The respective therapeutically effective amounts of mirtazapine and noscapine are as described above.

In some embodiments, the mirtazapine is substantially pure R-configuration mirtazapine, i.e., substantially pure (R)-mirtazapine.

According to yet another aspect of the present invention, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat organ fibrosis related diseases in a patient in need thereof, wherein the organ fibrosis related disease is selected from one or more of fibrosing interstitial lung diseases, renal interstitial fibrosis diseases, liver fibrosis diseases, and myocardial fibrosis diseases.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat progressive fibrosing interstitial lung disease in a patient in need thereof.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat progressive pulmonary fibrosis in a patient in need thereof.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat idiopathic pulmonary fibrosis in a patient in need thereof.

In some embodiments, a pharmaceutical kit is provided, comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat pneumoconiosis, reduce its severity, or prevent its progression in a patient in need thereof.

In some embodiments, the pharmaceutically acceptable salt of noscapine is noscapine hydrochloride.

In some embodiments, the mirtazapine is substantially pure R-configuration mirtazapine, i.e., substantially pure (R)-mirtazapine.

Those skilled in the art will understand that the above-described technical features of the pharmaceutical composition comprising mirtazapine or a pharmaceutically acceptable salt thereof and a pharmaceutically acceptable excipient, as well as the technical features of the pharmaceutical composition comprising noscapine or a pharmaceutically acceptable salt thereof and a pharmaceutically acceptable excipient, are applicable to the pharmaceutical kit of the present invention.

The pharmaceutical compositions of the present invention can be prepared into unit dosage forms for administration. The dosage forms may be liquid dosage forms or solid dosage forms. Liquid dosage forms may include solutions, colloids, emulsions, or suspensions, etc. Solid dosage forms may include, for example, tablets, powders, suppositories, granules, or capsules, etc. Other dosage forms include aerosols, implants, patches, or liniments, etc.

The pharmaceutical compositions of the present invention can be prepared into dosage forms suitable for use in patients using methods known in the art and appropriate pharmaceutical excipients. The selection of specific excipients will depend on the route of administration used to treat a specific patient or the type and condition of the disease. For example, pharmaceutically acceptable excipients may include diluents, carriers, fillers, binders, wetting agents, disintegrants, absorption enhancers, surfactants, adsorbent carriers, and lubricants, etc. that are conventional in the pharmaceutical field. If necessary, flavoring agents, preservatives, and sweeteners, etc., may also be added to the pharmaceutical compositions. Methods for preparing suitable pharmaceutical compositions for specific administration modes are well within the knowledge of those skilled in the pharmaceutical art.

The pharmaceutical compositions of the present invention can be administered by any method and in any form commonly used in the art. For example, the pharmaceutical compositions of the present invention may be administered via routes selected from: oral administration, spray inhalation, rectal administration, nasal administration, vaginal administration, topical administration, parenteral administration such as subcutaneous, intravenous, intramuscular, intraperitoneal, intrathecal, intraventricular, intrasternal, or intracranial injection. Among these, oral administration, intramuscular injection, intraperitoneal injection, or intravenous injection are preferred. Preferably, the pharmaceutical compositions of the present invention are administered to a patient in need thereof via oral administration.

For oral administration, any appropriate pharmaceutical excipients commonly used in the preparation of solid preparations can be used, and the pharmaceutical compositions of the present invention can be formulated into solid preparations, e.g., tablets, capsules, and powders, using formulation techniques known in the art. Alternatively, any appropriate pharmaceutical excipients commonly used in the preparation of liquid preparations can be used, and the pharmaceutical compositions of the present invention can be formulated into liquid preparations, e.g., solutions, syrups, suspensions, and emulsions, using formulation techniques known in the art.

For parenteral administration, any appropriate pharmaceutical excipients commonly used in the preparation of preparations suitable for parenteral administration can be used, and the pharmaceutical compositions of the present invention can be formulated into preparations suitable for parenteral administration, e.g., sterile injectables, lyophilized powders, transdermal patches, aerosols, oral and nasal inhalants, and suppositories, using formulation techniques known in the art. Parenteral administration routes include intravenous, intraperitoneal, subcutaneous, intramuscular, transdermal, nasal, intrapulmonary, intrathecal, rectal, and topical routes, etc.

Using formulation techniques known in the art, by appropriately adjusting the effective amount of the compound of the present invention, the dosage form, and/or various pharmaceutical additives, the pharmaceutical compositions can also be prepared into pharmaceutical formulations suitable for children, the elderly, critically ill patients, or surgical patients.

Based on the blood drug concentration observed when antifibrotic effects are achieved in animal tests, and with reference to the ratio between the dose at which antidepressant effects are observed and the dose at which antifibrotic effects are observed in animal tests, the effective dose range of mirtazapine for antifibrosis can be deduced from the dose range at which antidepressant effects are observed after administration in humans. When the subject is a human, the therapeutically effective amount of mirtazapine applicable in the present invention in a single dose is 0.1 to 45 mg, 0.5 to 30 mg, 0.5 to 15 mg, 0.5 to 10 mg, 1 to 10 mg, or 3 to 5 mg, e.g., 0.1 mg, 0.5 mg, 1 mg, 2 mg, 2.5 mg, 3 mg, 3.5 mg, 3.75 mg, 4 mg, 4.5 mg, 5 mg, 10 mg, 15 mg, 20 mg, 25 mg, 30 mg, or 45 mg, administered once or multiple times a day, preferably once a day. Based on the therapeutically effective amount of mirtazapine, those skilled in the art can easily determine the therapeutically effective amount of a pharmaceutically acceptable salt of mirtazapine.

When the subject is a human, the therapeutically effective amount of noscapine applicable in the present invention in a single dose is 1 to 3000 mg, 1 to 2000 mg, 1 to 1000 mg, 5 to 1000 mg, 10 to 1000 mg, 10 to 500 mg, 10 to 200 mg, or 10 to 120 mg, preferably 10 to 120 mg, e.g., 10 mg, 20 mg, 30 mg, 40 mg, 50 mg, 60 mg, 70 mg, 80 mg, 90 mg, 100 mg, 110 mg, or 120 mg, administered once or multiple times a day, preferably once a day. Based on the therapeutically effective amount of noscapine, those skilled in the art can easily determine the therapeutically effective amount of a pharmaceutically acceptable salt of noscapine.

Unless otherwise specifically stated in the present specification, the doses, dose ratios, contents, or content ratios of mirtazapine and noscapine disclosed in the present application are calculated based on their respective free forms rather than their salt or hydrate forms.

### Examples

Exemplary methods and materials are described in the present application, although methods and materials similar or equivalent to those described herein can also be used in the implementation or testing of various aspects and embodiments. The materials, methods, and examples specifically disclosed in the present application are for illustrative purposes only and are not intended to limit the scope of the present invention.

### Example 1: Establishment of a bleomycin-induced pulmonary fibrosis mouse model and evaluation of drug effects

This model is a classic one for evaluating the effect of substances on pulmonary fibrosis, and its establishment method is known to those skilled in the art.

Experimental animals: C57BL/6J mice, 8 weeks old, male.

Preparation of bleomycin solution: Bleomycin sulfate was weighed and put into a clean test tube, normal saline was then added to prepare a 0.32 mg/mL solution, and the solution was vortexed until it became clear.

### Method for establishing the bleomycin-induced pulmonary fibrosis mouse model

All mice were anesthetized with Zoletil/Xylazine (50 mpk/10 mpk; I.P.). On the first day of the experiment, mice in the model group were given an intratracheal injection of bleomycin hydrochloride with a volume of 50 µL. Mice in the sham operation group were given an intratracheal injection of normal saline with a volume of 50 µL. The drug administration period started on the 5^{th} day of the experiment and lasted until the 21^{st} day of the experiment.

Collection of lung tissues, and quantitative determination of fibrotic area by Masson's trichrome staining:
At the end of the study (the 21^{st} day of the experiment), all animals were euthanized 2 hours after the last drug administration. After perfusing all animals with normal saline, the left lung of each animal was collected and fixed in a fixative containing 10% neutral formalin for histological analysis. Collagen in lung tissues was quantified by Masson's trichrome staining, as described below:
Lung sections with a thickness of 4 µm were dried in an oven for 1 hour and then subjected to standardized Masson's trichrome staining. The steps were as follows: staining with Weigert's hematoxylin working solution for 10 minutes, then rinsing with water; staining with a mixed solution of Biebrich scarlet and fuchsin for 10 minutes, then rinsing with water; staining in a mixed solution of 5% phosphomolybdic acid and 5% tungstic acid for 5 minutes or until collagen no longer appeared red; immersing the glass slides in aniline blue solution, staining for 1 minute, then dedifferentiating in 1% acetic acid solution; subsequently, conducting gradient dehydration and covering the slides with coverslips. The stained sections were scanned using Aperio GT450 at 200× magnification, and then the percentage of fibrosis (positive area) in the selected annotations was calculated using HALO program. The fibrosis of each lung section was expressed as a percentage.

Before the experiment, the mice were acclimated to the environment for 1 week. Each mouse underwent a health check, including evaluation of fur, limbs, and mouthparts, and inspection for any abnormal signs in posture and movement.

### Example 2: Evaluation of the effects of mirtazapine administered alone or in combination with simvastatin in the bleomycin-induced pulmonary fibrosis mouse model

Preparation of vehicle (0.5% methylcellulose, w/w): Methylcellulose was weighed, normal saline was added, and, after stirring vigorously, the vehicle obtained was stored at 4°C.

Preparation of mirtazapine solution: Mirtazapine was weighed and put into the vehicle, then normal saline was added to prepare a 0.5 mg/mL solution, and the solution was vortexed and sonicated until it became clear. The administration dose was 10 mL/kg.

Preparation of simvastatin solution: Simvastatin was weighed and put into the vehicle, then normal saline was added to prepare a 0.2 mg/mL solution, and the solution was vortexed and sonicated until it became clear. The administration dose was 10 mL/kg.

Preparation of mirtazapine + simvastatin combined solution: Mirtazapine and simvastatin were weighed and put into the vehicle, a solution containing 0.5 mg/mL mirtazapine and 0.2 mg/mL simvastatin was prepared, and the solution was vortexed and sonicated until it became clear. The administration dose was 10 mL/kg.

**Table 1. Administration composition of mirtazapine alone or in combination with simvastatin**

| Group | Treatment (oral) | Number of mice |
|---|---|---|
| 1 | Vehicle | 10 |
| 2 | Mirtazapine 5 mg/kg | 10 |
| 3 | Simvastatin 2 mg/kg + mirtazapine 5 mg/kg | 10 |
| 4 | Simvastatin 2 mg/kg | 10 |

### Experimental results

According to the administration dose and route shown in Table 1, animals were administered following the method described in Example 1. The results obtained indicated that mirtazapine administered alone reduced the fibrotic area by 10.8%, simvastatin administered alone reduced the fibrotic area by 8.3%, while the combination of mirtazapine and simvastatin only reduced the fibrotic area by 15.3%, being less than the sum of their individual antifibrotic effects.

These results demonstrate that both mirtazapine and simvastatin exhibit a tendency to reduce the fibrotic area. However, when the antifibrotic effect of the combination of mirtazapine and simvastatin is compared with that of each drug administered alone, it can be seen that the antifibrotic efficacy of the combination does not show an enhancement.

### Example 3: Evaluation of the effects of mirtazapine administered alone or in combination with noscapine in the bleomycin-induced pulmonary fibrosis mouse model

Preparation of vehicle (0.5% methylcellulose, w/w): Methylcellulose was weighed, normal saline was added, and, after stirring vigorously, the vehicle obtained was stored at 4°C.

Preparation of mirtazapine solution: Mirtazapine was weighed and put into the vehicle, then normal saline was added to prepare a 0.5 mg/mL solution, and the solution was vortexed and sonicated until it became clear. The administration dose was 10 mL/kg.

Preparation of noscapine solution: Noscapine hydrochloride was weighed and put into the vehicle, then normal saline was added to prepare a 3 mg/mL solution, and the solution was vortexed and sonicated until it became clear. The administration dose was 10 mL/kg.

Preparation of mirtazapine + noscapine combined solution: Mirtazapine and noscapine hydrochloride were weighed and put into the vehicle, a solution containing 0.5 mg/mL mirtazapine and 3 mg/mL noscapine was prepared, and the solution was vortexed and sonicated until it became clear. The administration dose was 10 mL/kg.

**Table 2. Administration composition of mirtazapine alone or in combination with noscapine**

| Group | Treatment (oral) | Number of mice |
|---|---|---|
| 1 | Vehicle | 8 |
| 2 | Mirtazapine 5 mg/kg | 8 |
| 3 | Noscapine 30 mg/kg + mirtazapine 5 mg/kg | 8 |
| 4 | Noscapine 30 mg/kg | 8 |

### Experimental results

According to the administration dose and route shown in Table 2, animals were administered in accordance with the method described in Example 1. The results obtained indicated that mirtazapine administered alone reduced the fibrotic area by 14.3%, noscapine administered alone reduced the fibrotic area by 3.3%, while the combination of mirtazapine and noscapine unexpectedly reduced the fibrotic area by 20.5%, being greater than the sum of their individual antifibrotic effects, and the extent of reduction was statistically significant.

These results demonstrate that mirtazapine alone exhibits a tendency to reduce the fibrotic area, whereas noscapine alone fails to reduce the fibrotic area. However, when noscapine, for which no antifibrotic effect is observed when administered alone, is combined with mirtazapine, the antifibrotic effect of the combination is found to be greater than the sum of their individual antifibrotic effects, i.e., a synergistic effect is achieved.

Therefore, the inventors have experimentally confirmed that when certain compounds that exhibit antifibrotic effects when used alone are administered in combination, the combined administration does not show an enhancement of the antifibrotic effect. This indicates that the antifibrotic effect of drug combinations is unpredictable. Surprisingly, when the inventors administer noscapine, which does not exhibit an antifibrotic effect when used alone, in combination with mirtazapine, the combined administration shows a synergistic enhancement of the antifibrotic effect. The synergistic antifibrotic effect of the combination of mirtazapine and noscapine is unexpected to those skilled in the art.

### Example 4: Evaluation of the effect of (R)-mirtazapine in a bleomycin-induced pulmonary fibrosis mouse model

Preparation of bleomycin solution: Bleomycin sulfate was weighed and put into a clean test tube, normal saline was then added to prepare a 0.32 mg/mL solution, and the solution was vortexed until it became clear.

Preparation of vehicle (0.5% methylcellulose, w/w): Methylcellulose was weighed, normal saline was added, and, after stirring vigorously, the vehicle obtained was stored at 4°C.

Preparation of mirtazapine solution: (S)-mirtazapine or (R)-mirtazapine was weighed and put into the vehicle, then normal saline was added to prepare a 0.5 mg/mL solution, and the solution was vortexed and sonicated until it became clear. The administration dose was 10 mL/kg.

**Table 3. Administration composition of (S)-mirtazapine and (R)-mirtazapine**

| Group | Treatment (oral) | Number of mice |
|---|---|---|
| 1 | Model vehicle | 8 |
| 2 | (S)-mirtazapine 5 mg/kg | 8 |
| 3 | (R)-mirtazapine 5 mg/kg | 8 |

### Method for establishing the bleomycin-induced pulmonary fibrosis mouse model

Before the experiment, the mice were acclimated to the environment for 1 week. Each mouse underwent a health check, including evaluation of fur, limbs, and mouthparts, and inspection for any abnormal signs in posture and movement.

All mice were anesthetized with Zoletil/Xylazine (50 mpk/10 mpk; I.P.). On the first day of the experiment, mice in all three groups were given an intratracheal injection of bleomycin hydrochloride with a volume of 50 µL. The drug administration period started on the 5^{th} day of the experiment and lasted until the 21^{st} day of the experiment, with oral administration once a day for 17 consecutive days.

### Experimental results

According to the administration dose and route shown in Table 3, animals were administered in accordance with the method described in Example 4. The results obtained indicated that the (S)-mirtazapine group reduced the fibrotic area by 18.2%, whereas the (R)-mirtazapine group reduced the fibrotic area by 25.1%, and moreover, a statistically significant reduction in the fibrotic area was only observed in the (R)-mirtazapine group. It was (R)-mirtazapine, rather than (S)-mirtazapine that is considered the main active form for antidepressant effects, that exhibited a more pronounced antifibrotic effect. This result is unexpected to those skilled in the art.

### Example 5: Clinical evaluation of the effects of mirtazapine administered alone or in combination with noscapine in patients with idiopathic pulmonary fibrosis

To evaluate the efficacy of mirtazapine administered alone or in combination with noscapine in patients with idiopathic pulmonary fibrosis, the following clinical study will be conducted.

Taking approximately 100 patients diagnosed with idiopathic pulmonary fibrosis within 5 years according to the ATS/ERS/JRS/ALAT international diagnostic guidelines and/or the updated guidelines (2022) as subjects, the efficacy of mirtazapine administered alone or in combination with noscapine will be verified through clinical and biological monitoring. The subjects will take oral doses of mirtazapine tablets alone, noscapine tablets alone, or a combination of mirtazapine tablets and noscapine tablets. The doses will be as follows: mirtazapine 3 to 5 mg once daily (for example, if 15 mg mirtazapine tablets are used, a 1/4 tablet may be administered) and noscapine 20 mg once daily. The medication will be taken continuously for 24 weeks. For the assessment of idiopathic pulmonary fibrosis symptoms, changes in symptoms after administration can be objectively evaluated by measuring indicators such as forced vital capacity (FVC) and diffusing capacity of the lungs for carbon monoxide (DLCO), or by imaging assessment using high-resolution computed tomography (HRCT).

## Claims

1. A pharmaceutical composition comprising mirtazapine or a pharmaceutically acceptable salt thereof, noscapine or a pharmaceutically acceptable salt thereof, and a pharmaceutically acceptable excipient.

2. The pharmaceutical composition according to claim 1, which is in the form of dosage units, wherein each dosage unit contains mirtazapine at a dose in the range of 0.1 to 45 mg, 0.5 to 15 mg, 0.5 to 10 mg, 1 to 10 mg, or 3 to 5 mg, or a corresponding amount of a pharmaceutically acceptable salt of mirtazapine.

3. The pharmaceutical composition according to claim 1 or 2, which is in the form of dosage units, wherein each dosage unit contains noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, or a corresponding amount of a pharmaceutically acceptable salt of noscapine.

4. The pharmaceutical composition according to any one of claims 1-3, wherein the mirtazapine is substantially pure (R)-mirtazapine.

5. Use of a combination of mirtazapine or a pharmaceutically acceptable salt thereof and noscapine or a pharmaceutically acceptable salt thereof in the manufacture of a medicament for treating an organ fibrosis related disease in a patient in need thereof.

6. The use according to claim 5, wherein the organ fibrosis related disease is selected from one or more of fibrosing interstitial lung diseases, renal interstitial fibrosis diseases, liver fibrosis diseases, and myocardial fibrosis diseases.

7. The use according to claim 5, wherein the organ fibrosis related disease is progressive fibrosing interstitial lung disease, progressive pulmonary fibrosis, or idiopathic pulmonary fibrosis.

8. The use according to any one of claims 5-7, wherein the manufactured medicament contains mirtazapine at a dose in the range of 0.1 to 45 mg, 0.5 to 15 mg, 0.5 to 10 mg, 1 to 10 mg, or 3 to 5 mg, or a corresponding amount of a pharmaceutically acceptable salt of mirtazapine.

9. The use according to any one of claims 5-8, wherein the manufactured medicament contains noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, or a corresponding amount of a pharmaceutically acceptable salt of noscapine.

10. Use of a combination of mirtazapine or a pharmaceutically acceptable salt thereof and noscapine or a pharmaceutically acceptable salt thereof in the manufacture of a medicament for improving pulmonary fibrosis lesions, inhibiting the progression of pulmonary fibrosis, or reducing the severity of pulmonary fibrosis in a patient.

11. The use according to any one of claims 5-10, **characterized in that** the mirtazapine or pharmaceutically acceptable salt thereof and the noscapine or pharmaceutically acceptable salt thereof can be formulated into a single pharmaceutical formulation.

12. The use according to any one of claims 5-10, **characterized in that** the mirtazapine or pharmaceutically acceptable salt thereof and the noscapine or pharmaceutically acceptable salt thereof can each be formulated into separate pharmaceutical formulations for simultaneous or sequential administration.

13. The use according to any one of claims 5-12, wherein the mirtazapine is substantially pure (R)-mirtazapine.

14. A pharmaceutical kit comprising: (1) a pharmaceutical composition comprising mirtazapine or a pharmaceutically acceptable salt thereof and a pharmaceutically acceptable excipient; (2) a pharmaceutical composition comprising noscapine or a pharmaceutically acceptable salt thereof and a pharmaceutically acceptable excipient; and (3) instructions for administrating the pharmaceutical compositions to treat organ fibrosis related diseases in a patient in need thereof.

15. The pharmaceutical kit according to claim 14, wherein the pharmaceutical composition (1) comprises mirtazapine at a dose in the range of 0.1 to 45 mg, 0.5 to 15 mg, 0.5 to 10 mg, 1 to 10 mg, or 3 to 5 mg, or a corresponding amount of a pharmaceutically acceptable salt of mirtazapine.

16. The pharmaceutical kit according to claim 14 or 15, wherein the pharmaceutical composition (2) comprises noscapine at a dose in the range of 1 to 3000 mg, 1 to 2000 mg, 1 to 1000 mg, 5 to 1000 mg, 10 to 1000 mg, 10 to 500 mg, 10 to 200 mg, or 10 to 120 mg, or a corresponding amount of a pharmaceutically acceptable salt of noscapine.

17. The pharmaceutical kit according to any one of claims 14-16, wherein the organ fibrosis related disease is progressive fibrosing interstitial lung disease, progressive pulmonary fibrosis, or idiopathic pulmonary fibrosis.

18. The pharmaceutical kit according to any one of claims 14-17, wherein the mirtazapine is substantially pure (R)-mirtazapine.

19. A method for treating an organ fibrosis related disease in a patient, comprising a step of administering to the patient mirtazapine at a dose in the range of 0.1 to 45 mg, 0.5 to 30 mg, 0.5 to 15 mg, 0.5 to 10 mg, 1 to 10 mg, or 3 to 5 mg, or a corresponding amount of a pharmaceutically acceptable salt of mirtazapine, once or multiple times a day.

20. The method according to claim 19, wherein the organ fibrosis related disease is selected from one or more of fibrosing interstitial lung diseases, renal interstitial fibrosis diseases, liver fibrosis diseases, and myocardial fibrosis diseases.

21. The method according to claim 19 or 20, comprising administering to the patient mirtazapine at a daily dose of less than 15 mg, or a corresponding amount of a pharmaceutically acceptable salt of mirtazapine.

22. The method according to any one of claims 19-21, wherein the mirtazapine is substantially pure (R)-mirtazapine.

23. A method for improving pulmonary fibrosis lesions, inhibiting the progression of pulmonary fibrosis, or reducing the severity of pulmonary fibrosis in a patient, comprising a step of administering to the patient substantially pure mirtazapine at a dose in the range of 0.1 to 45 mg, 0.5 to 30 mg, 0.5 to 15 mg, 0.5 to 10 mg, 1 to 10 mg, or 3 to 5 mg, or a corresponding amount of a pharmaceutically acceptable salt of mirtazapine, once or multiple times a day.

24. The method according to claim 23, comprising administering to the patient mirtazapine at a daily dose of less than 15 mg, or a corresponding amount of a pharmaceutically acceptable salt of mirtazapine.

25. The method according to claim 23 or 24, wherein the mirtazapine is substantially pure (R)-mirtazapine.

26. A method for treating an organ fibrosis related disease in a patient, comprising a step of administering to the patient a therapeutically effective amount of substantially pure (R)-mirtazapine or a pharmaceutically acceptable salt thereof.

27. The method according to claim 26, wherein the organ fibrosis related disease is selected from one or more of fibrosing interstitial lung diseases, renal interstitial fibrosis diseases, liver fibrosis diseases, and myocardial fibrosis diseases.

28. The method according to claim 26 or 27, wherein the therapeutically effective amount of the substantially pure (R)-mirtazapine is any dose in the range of 0.1 to 45 mg, 0.5 to 30 mg, 0.5 to 15 mg, 0.5 to 10 mg, 1 to 10 mg, or 3 to 5 mg.

29. Use of substantially pure (R)-mirtazapine or a pharmaceutically acceptable salt thereof in the manufacture of a medicament for treating an organ fibrosis related disease in a patient.

30. The use according to claim 29, wherein the organ fibrosis related disease is selected from one or more of fibrosing interstitial lung diseases, renal interstitial fibrosis diseases, liver fibrosis diseases, and myocardial fibrosis diseases.

31. Use of substantially pure (R)-mirtazapine or a pharmaceutically acceptable salt thereof in the manufacture of a medicament for improving pulmonary fibrosis lesions, inhibiting the progression of pulmonary fibrosis, or reducing the severity of pulmonary fibrosis in a patient.

32. A method for improving pulmonary fibrosis lesions, inhibiting the progression of pulmonary fibrosis, or reducing the severity of pulmonary fibrosis in a patient, comprising a step of administering to the patient an effective amount of substantially pure (R)-mirtazapine or a pharmaceutically acceptable salt thereof.

33. The method according to claim 32, wherein the therapeutically effective amount of the substantially pure (R)-mirtazapine is any dose in the range of 0.1 to 45 mg, 0.5 to 30 mg, 0.5 to 15 mg, 0.5 to 10 mg, 1 to 10 mg, or 3 to 5 mg.

34. A pharmaceutical composition comprising substantially pure (R)-mirtazapine or a pharmaceutically acceptable salt thereof and a pharmaceutically acceptable excipient, for treating an organ fibrosis related disease in a patient.

35. The pharmaceutical composition for use according to claim 34, wherein the organ fibrosis related disease is selected from one or more of fibrosing interstitial lung diseases, renal interstitial fibrosis diseases, liver fibrosis diseases, and myocardial fibrosis diseases.

36. A pharmaceutical composition comprising substantially pure (R)-mirtazapine or a pharmaceutically acceptable salt thereof and a pharmaceutically acceptable excipient, for improving pulmonary fibrosis lesions, inhibiting the progression of pulmonary fibrosis, or reducing the severity of pulmonary fibrosis in a patient.

37. Substantially pure (R)-mirtazapine or a pharmaceutically acceptable salt thereof, for use in improving pulmonary fibrosis lesions, inhibiting the progression of pulmonary fibrosis, or reducing the severity of pulmonary fibrosis in a patient.

38. Substantially pure (R)-mirtazapine or a pharmaceutically acceptable salt thereof, for use in treating an organ fibrosis related disease in a patient.

39. The substantially pure (R)-mirtazapine or a pharmaceutically acceptable salt thereof for use according to claim 38, wherein the organ fibrosis related disease is selected from one or more of fibrosing interstitial lung diseases, renal interstitial fibrosis diseases, liver fibrosis diseases, and myocardial fibrosis diseases.

40. A pharmaceutical composition comprising substantially pure (R)-mirtazapine or a pharmaceutically acceptable salt thereof and a pharmaceutically acceptable excipient.
